**Europäisches Patentamt**

(19) **European Patent Office**

**Office européen des brevets**

(11) **EP 0 637 219 B1**

(12) ## EUROPEAN PATENT SPECIFICATION

(45) Date of publication and mention
of the grant of the patent:
**04.11.1998 Bulletin 1998/45**

(21) Application number: **92915443.3**

(22) Date of filing: **07.02.1992**

(51) Int. Cl.$^6$: **A61B 5/02**, A61B 5/028

(86) International application number:
**PCT/US92/01012**

(87) International publication number:
**WO 93/15652 (19.08.1993 Gazette 1993/20)**

(54) **A THERMODILUTION CATHETER HAVING A SAFE, FLEXIBLE HEATING ELEMENT**

WÄRMEVERDÜNNUNGSKATHETER MIT BIEGSAMEM, SICHEREM HEIZELEMENT

CATHETER DE THERMODILUTION POURVU D'UN ELEMENT CHAUFFANT FLEXIBLE FIABLE

(84) Designated Contracting States:
**DE FR GB**

(43) Date of publication of application:
**08.02.1995 Bulletin 1995/06**

(73) Proprietor:
**BAXTER INTERNATIONAL INC.
Deerfield, IL 60015-4633 (US)**

(72) Inventors:
• **QUINN, Michael, D.
Plano, TX 75093 (US)**
• **YELDERMAN, Mark, L.
Plano, TX 75093 (US)**

(74) Representative:
**MacGregor, Gordon et al
Eric Potter Clarkson,
Park View House,
58 The Ropewalk
Nottingham NG1 5DD (GB)**

(56) References cited:
| | |
|---|---|
| EP-A- 0 182 363 | US-A- 3 075 515 |
| US-A- 3 798 967 | US-A- 4 073 287 |
| US-A- 4 240 441 | US-A- 4 499 907 |
| US-A- 4 632 125 | US-A- 4 841 981 |
| US-A- 4 856 530 | US-A- 4 901 734 |
| US-A- 5 056 526 | US-A- 5 056 526 |
| US-A- 5 217 019 | |

EP 0 637 219 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

# Description

## BACKGROUND OF THE INVENTION

### Field of the Invention

The present invention relates to a thermodilution catheter having a flexible heating filament disposed therein for applying heat to a patient's blood for purposes of measuring cardiac output, volumetric blood flow, blood pressure, blood volume, blood components, or the like.

### Description of the Prior Art

As is well known, catheters have been developed for measuring cardiovascular parameters such as cardiac output, blood pressure, blood volume, blood components and the like. Conventional catheters are made from various materials including plastics and are typically inserted into various body compartments, cavities and vessels to either deliver therapeutic agents, diagnostic agents, or to measure directly various physiologic parameters.

Numerous techniques have been disclosed in the prior art for measuring blood flow using catheters. For example, in U.S. Patent No. 4,507,974, Yelderman describes a technique for measuring blood flow by applying a stochastic excitation signal to a system inlet and measuring the output signal at a downstream system outlet. The blood flow rate is then extracted by cross-correlating the excitation signal and the measured output signal. The problem addressed by systems of this type is particularly difficult since the physiologic blood vessels are elastic, thereby making classic fluid measuring techniques unacceptably inaccurate. In fact, because the blood vessels are elastic, blood flow cannot be measured unless (1) the physical heart dimensions are measured simultaneously with the blood velocity, (2) a technique is used which is independent of the vessel geometry or (3) a blood velocity technique is used which is calibrated by some other technique. Examples of each of these techniques may be found in the prior art.

For example, a prior art approach for simultaneously measuring blood velocity and vessel geometry is described by Segal in U.S. Patent Nos. 4,733,669 and 4,869,263 and in an article entitled "Instantaneous and Continuous Cardiac Output Obtained With a Doppler Pulmonary Artery Catheter", Journal of the American College of Cardiology, Vol. 13, No. 6, May 1989, Pages 1382-1392. Segal therein discloses a Doppler pulmonary artery catheter system which provides instantaneous diameter measurements and mapping of instantaneous velocity profiles within the main pulmonary artery from which instantaneous cardiac output is calculated. A similar approach is taught by Nassi et al. in U.S. Patent No. 4,947,852. A comparable ultrasound technique is disclosed by Abrams, et al. in U.S. Patent Nos. 4,671,295 and 4,722,347 and in an article entitled "Transtracheal Doppler: A New Procedure for Continuous Cardiac Output Measurement", Anesthesiology, Vol. 70, No. 1, January 1989, Pages 134-138. Abrams et al. therein describe a technique whereby a piezoelectric ultrasound transducer is placed in the trachea of a patient in proximity to the aorta or pulmonary artery so that ultrasound waves may be transmitted toward the path of flow of blood in the artery and reflected waves received. The cross-sectional size of the artery is measured based upon the Doppler frequency difference between the transmitted and received waves. Imaging techniques such as x-ray or radio isotope methods have also been used.

Previous techniques which are geometry independent include an indicator dilution or dye dilution technique of the type first disclosed by Stewart in an article entitled "The Output of the Heart in Dogs", American Journal of Physiology, Vol. 57, 1921, Pages 27-50. Other such geometry independent techniques include a thermodilution technique as first described by Fegler in an article entitled "Measurement of Cardiac Output in Anesthetized Animals by a Thermo-Dilution Method", Quarterly Journal of Experimental Physiology, Vol. 39, 1954, Pages 153-164 and an ionic dilution technique as described by Geddes et al. in U.S. Patent No. 4,572,206.

On the other hand, prior art techniques for measuring blood velocity which require a secondary calibration technique include a pulse contour technique of the type described by Schreuder, et al. in an article entitled "Continuous Cardiac Output Monitoring During Cardiac Surgery", Update In Intensive Care And Emergency Medicine, Berlin: Springer-Verlag, 1990, Pages 413-416. Another so-called "hot wire" anemometer or heated thermistor technique has been described, for example, by Tanabe, et al. in U.S. Patent No. 4,841,981 and EP 235811 and by Sekii, et al. in U.S. Patent No. 4,685,470 and WO 8806426.

The present invention relates to a geometry independent technique, namely, indicator dilution. In conventional indicator dilution techniques, different methods of indicator delivery may be used. For example, Khalil in U.S. Patent No. 3,359,974 introduces indicator as a step increase and measures the resultant distal temperature change. Newbower, et al., on the other hand, discloses in U.S. Patent No. 4,236,527 the technique of introducing the indicator as a sinusoid and measuring the distal wave attenuation. In addition, the indicator may be applied as an impulse so that the area under the resultant response may be measured as described by Normann in U.S. Patent No. 4,576,182. Eggers, et al. in U.S Patent No. 4,785,823 similarly provide an impulse, but Eggers, et al. use high frequency energy to provide large heat fluxes to the blood without increasing the filament temperature. In addition, Petre describes in U.S. Patent No. 4,951,682 an intra-cardiac impedance catheter which measures cardiac output based on changes in the electrical impedance of the blood in the right ventricle. By contrast, as described by Yelderman in the afore-mentioned U.S. Patent No. 4,507,974, the indicator may be supplied according to a pseudo-random binary sequence and the distal response measured. Cross-correlation can then be performed between the input sequence and the output sequence, and flow is computed based upon the area under the cross-correlation curve. Each of these techniques may provide either an intermittent or a continuous measurement.

Although each of the above-mentioned techniques may use a variety of indicators, heat is the preferred indicator to be used in the clinical environment, for unlike other indicators, heat is conserved in the immediate vascular system but is largely dissipated in the periphery in one circulation time so as to eliminate recirculation and accumulation problems. On the other hand, if cold (negative heat) indicators are used, large amounts of cold may be used, for cold has relatively no deleterious effects on blood and surrounding tissues. However, when cold is used, it must be supplied in a fluid carrier such as saline since cold producing transducers are not readily economical or technically available at present. For example, such a technique is described by Webler in U.S. Patent No. 4,819,655 and by Williams in U.S. Patent No. 4,941,475, but the cold-based technique of Webler or Williams has significant clinical limitations in that the circulating fluid must be cooled to near ice temperature prior to input into the catheter and temperature equilibrium established, which takes a significant amount of time. In addition, the enlarged catheter segment containing the cooling elements may restrict blood flow. By contrast, if heat is used, a maximum heat infusion limitation is quickly reached since small increases in heat transducer temperature can have a deleterious effect on blood and local tissue. In fact, it can be inferred from the teachings of Ham et al. in "Studies in Destruction of Red Blood Cells, Chapter IV. Thermal Injury", Blood, Vol. 3, pp. 373-403 (1948), by Ponder in "Shape and Transformations of Heated Human Red Cells", J. Exp. Biol., Vol. 26, pp. 35-45 (1950) and by Williamson et al. in "The Influence of Temperature on Red Cell Deformability", Blood, Vol. 46, pp. 611-624 (1975), that a maximum safe filament surface temperature is probably about 48°C. Since the surface temperature of a heat transducer is a function of the blood flow velocity, the surface area and the heat flux, the optimum design is to maximize the heat delivered to the blood while minimizing the transducer surface temperature.

A heat transducer must satisfy several requirements if it is to be used clinically. Namely, the heat transducer or filament must be electrically safe. It also must only minimally increase the catheter cross-sectional area or diameter and must be made of materials which are non-toxic, which can be sterilized, and which can safely and easily pass through a standard introducer sheath. The heater must also be flexible so as not to increase the stiffness of the catheter body and must be capable of transferring the electrically generated heat to the surrounding blood without exceeding a safe filament surface temperature. Moreover, means must be present to continuously monitor the filament temperature to detect unsafe filament temperature and/or stagnant blood flow. However, prior art heater elements for catheters have not heretofore addressed these problems.

Prior art heater elements for thermodilution catheters have typically used simple resistive wire wound around the catheter. For example, Khalil discloses in U.S. Patent No. 3,359,974 and Barankay, et al. disclose in an article entitled "Cardiac Output Estimation by a Thermodilution Method Involving Intravascular Heating and Thermistor Recording", Acta Physiologica Academiae Scientiarum Hungaricae, Tomus 38 (2-3), 1970, Pages 167-173, wrapping the wire around the catheter but describe no methods for securing the heater material to the catheter body. Normann, in U.S. Patent No. 4,576,182, discloses a design similar to that of Khalil. However, such exposed or minimally required wire as used in these devices increases the catheter cross-section, thereby making it difficult for the catheter to pass through an introducer and providing a rough surface which may introduce local blood clot formation. In addition, such an arrangement provides no protection from fragments of the filament becoming dislodged and does not evenly dissipate the heat, thereby producing "hot" spots near the filament itself. It is thus desired to design a catheter heating filament which is electrically, mechanically and thermodynamically safe.

As noted above, although heat is a preferred indicator for dilution methods for measuring blood flow, the amount of heat delivered is limited or restricted by the maximal safe filament surface temperature. Although no absolute safe maximum filament temperature has been developed in the prior art, sufficient information is present in the literature to substantiate a reasonably safe maximum. For example, amongst blood, proteins and vessel tissue, red blood cells have been shown to be probably the most susceptible to higher temperatures. It is also well documented in the prior art that red blood cells can sustain an incubation temperature of 48°C for up to one hour before developing significant abnormalities, as described by Williamson, et al. In any event, because the actual contact time of each red blood cell flowing past the heating filament is significantly less than several seconds, such a maximum of 48°C is easily acceptable. A catheter heating filament can be designed to provide sufficient surface area to allow adequate heat infusion with a surface temperature below this maximum; however, changes in flow, such as sudden decreases or cardiac arrest, or changes in catheter position, such as becoming lodged against a vessel wall, may provide a local blood environment. Such stagnant environments may allow for surface temperatures which exceed these maximum limits and can thus cause harm if a method is not present for measuring filament temperature.

In addition, in prior art heating filaments for thermodilution catheters either the filament temperature has not been measured or the temperature is measured with a second thermometer. e.g. as disclosed in US-A-4240441 and US-A-4841981 Such techniques obviously are unacceptable if a maximum safe temperature is to be maintained. Acceptable temperature sensing materials require a sufficiently high temperature coefficient of resistance to measure changes in temperature. Compositions of this type are described for example, by Morris, et al. in an article entitled "Thin Film Thermistors", Journal of Physics Engineering: Scientific Instruments, Vol. 8, 1975, Pages 411-414. It is desired to develop a method for continuously measuring the filament temperature without the use of a secondary measuring transducer such as a thermistor or thermocouple of the type used in these prior art devices.

A classical prior art method of measuring fluid velocity uses a hot-wire anemometer. In accordance with this technique, a filament is heated with a constant power or at a constant heat flux and the resistance is measured. If a filament with a high temperature coefficient of resistance is used, the measured resistance can be used to directly calculate filament temperature, for the filament temperature is monotonically and inversely proportional to the fluid velocity. Such a technique has been previously described as a means for measuring blood velocity by Gibbs in an article entitled "A Thermoelectric Blood Flow Recorder in the Form of a Needle", Proc. Soc. Exp. Biol. & Med., Vol. 31, 1933, Pages 141-146. However, as noted by Gibbs in that article, such a technique has been limited to peripheral vessels and cannot give absolute blood volumetric flow rates, only velocity. It is desired to adapt such techniques to thermodilution measurements to prevent localized overheating of the blood.

As noted above, previous heating elements for thermodilution catheters have generally used wire and wrapped it around the catheter. However, such an approach provides for uneven heat densities on the catheter since the wire tends to be hot and the space between the wire cooler. A more uniform material is desired which allows for more even heat densities and the elimination of "hot" spots. This is not possible with wire, for wire, even very small gauge wire, provides a larger cross-section for the catheter than necessary since there is unused space between the wire even when the wire is wound very compactly. A more uniform material would allow for a better distribution of the same quantity of heat with only a small increase in catheter cross-section. It is thus desirable to develop a filament material which minimally increases a cross-sectional area of the catheter and which provides a more uniform filament heat flux.

Accordingly, there are numerous problems with prior art thermodilution catheters which render them either inaccurate or unacceptably unsafe for use in the clinical environment. There is thus a long-felt need in the art for a filamented thermodilution catheter which overcomes these limitations of the prior art so as to allow production of a safe, accurate thermodilution catheter.

The features of the present invention are set out in Claim 1, in which the precharacterising part is based on US-A-4240441.

A safe, accurate thermodilution measurement may be made by using a catheter having a heating filament which resides internal to the catheter body, either in a preformed catheter lumen or beneath an outer sheath, and which does not directly contact the blood. The heating filament is preferably made of a thin, flexible material which may be wrapped either on the exterior of the catheter body wall and then covered by an external sheath material so that the heating filament material is not exposed to the blood or on the outer surface of a supporting sheath inserted into the catheter lumen. The heating filament so designed supplies a quantity of heat to the flowing blood which is used for measuring the volumetric blood flow using an indicator dilution equation. The filament temperature is measured simultaneous with the thermodilution measurement without the use of a second measuring transducer - not to calculate velocity, but so that a safe filament temperature may be maintained. This is accomplished by forming the heating filament of a material which has a resistance proportional or inversely proportional to its temperature.

In addition, the invention allows determination of blood flow using thermodilution techniques in clinical situations which were impossible using the classic bolus thermodilution method. For example, in applications such as measuring left ventricular blood flow or measuring hepatic blood flow, the natural blood flow is "retro grade", i.e., the blood flows from the distal end of the catheter toward the proximal portion. In this situation, it is necessary to place the thermal filament at the distal tip and locate the detection thermistor or thermocouple in a proximal location. The classical thermodilution catheter cannot be used in such a case since it is necessary to have the indicator flow under and past the detection thermistor or thermocouple, which in the case of "retro grade" blood flow induces an enormous amount of cross-talk and error. However, if an electrical filament is used, such as in the present invention, the electrical current can be passed under and past the detection thermistor or thermocouple without inducing thermal cross-talk and error.

In accordance with a preferred embodiment of the invention, the catheter member comprises a substantially cylindrical body wall portion and an outer sheath, where the heating filament is wrapped in a thin layer about the body wall portion of the catheter member and enclosed by the sheath. The sheath may be a flexible material formed by extrusion or blow molding or a flexible material which shrinks to form-fit the heating filament and the body wall portion when a sufficient quantity of heat is applied thereto. A layer of material with high thermal conductivity may also be disposed about the heating filament so as to create a more uniform surface temperature. The body wall portion may also have a reduced diameter in the region where the heating filament is wrapped therearound such that the resulting total diameter in that region is approximately equal to the diameter of the body wall portion in other regions. In some embodiments, the sheath material may actually contain the resistive heating material so as to form one element, thereby eliminating the need for a separate filament and sheath.

The heating element is placed upstream or downstream from the temperature detecting means depending upon whether "retro grade" or flow-directed measurements are made. Also, at least one set of electrical leads is attached to the heating filament at a first end thereof so as to apply power to the heating filament and is attached at a second end thereof to a cardiac output computer which applies an appropriate power signal to the electrical leads when the predetermined quantity of heat is to be applied to the blood. Typically, the cardiac output computer continuously monitors both current and voltage delivered to the catheter filament. From the product of current and voltage, the delivered heat may be computed, and from the ratio of voltage to current, the actual filament temperature may be calculated.

Calibrating means may be provided for calibrating both the heating filament and the temperature detecting means. Preferably, the calibrating means comprises a Read Only Memory (ROM) contained within the catheter member for storing calibration information for at least one of the heating filament and the temperature detecting means, as well as any other necessary information. Such calibration information may further include heating filament resistance at a given temperature, heating filament heat transfer efficiency, temperature coefficient of resistance and thermistor information. Moreover, the ROM may be connected to a cardiac output computer so as to pass a program segment, stored in the ROM, of a program used by the cardiac output computer to calculate cardiac output of the patient, whereby calculation of the patient's cardiac output cannot commence until the cardiac output computer is connected to the ROM and the program segment transferred to the cardiac output computer.

The catheter member may have at least one lumen through which the heating filament is removably inserted. In such an embodiment, the heating filament is wrapped in a thin layer about a substantially cylindrical supporting member such that the combination has an outer diameter which is approximately equal to an inner diameter of the lumen through which it is inserted. Preferably, the heating filament is approximately 5 to 10 centimeters in length and is disposed approximately 10 centimeters from the temperature detecting means.

The heating filament is comprised of a material having a high temperature coefficient of resistivity, whereby resistance of the heating filament is inversely proportional to its temperature (i.e., it has a negative temperature coefficient of resistance). This aspect of the invention enables power to the heating filament to be reduced when resistance of the heating filament exceeds a predetermined resistance amount, which is reached when the temperature of the heating filament reaches approximately 52°C. A material suitable for the heating filament thus has a temperature coefficient of resistivity greater than 0.001 $\Omega/\Omega$-°C. Also, such a material preferably has a low thermal capacitance and high thermal conductivity. Preferred heating filament materials include an alloy of approximately 70% nickel and 30% iron and an alloy of approximately 29% nickel, 17% cobalt and 54% iron.

Thus, by providing a heating filament which is minimally thin, heat from the filament may be transferred to the surrounding blood without significantly increasing the overall cross-sectional area of the catheter. Also, by providing a surface coating for the resulting catheter which has no rough areas to induce blood clot formation, the thermodilution catheter of the invention may be more safely used in clinical settings. Moreover, by monitoring the heating filament temperature, localized overheating may be prevented. Other elements or drugs, such as heparin, antibiotics and the like may be added to the filament sheath to reduce the possibility of any complications as a result of the measurements using the catheter.

## BRIEF DESCRIPTION OF THE DRAWINGS

The above and other objects and advantages of the invention will become more apparent and more readily appreciated from the following detailed description of the presently preferred exemplary embodiments of the invention taken in conjunction with the accompanying drawings, of which:

FIG. 1 is an overall perspective view illustrating the proximal end of a catheter for measuring cardiac output in accordance with the present invention;

FIG. 2 illustrates a cross-sectional view of the catheter of FIG. 1 showing the filament lead lumen which receives the heating filament leads and/or heating element in accordance with the invention;

FIG. 3 illustrates a detail view of the heater connector 116 of the catheter of FIG. 1;

FIG. 4(a) illustrates a first embodiment of a distal end of the catheter of the invention for use in flow-directed measurement, whereby the heating filament is wound about a body wall portion of the catheter and is enclosed within an outer sheath;

FIG. 4(b) illustrates a modification of the first embodiment whereby the heating filament is flush with the adjacent section of the catheter body so as to prevent an increase in the catheter cross-section;

FIG. 5 illustrates a second embodiment of a distal end of the catheter of the invention for use in retro grade measurement, whereby the heating filament is wound about a body wall portion of the catheter and is enclosed within an outer sheath;

FIG. 6 illustrates a third embodiment of a distal end of the catheter of the invention for use in retro grade measurement, whereby a "pigtail" tip is provided to prevent blood vessel rupture;

FIG. 7 illustrates an embodiment whereby the heater element and its supporting sheath are inserted into the lumen of the catheter of FIG. 1; and

FIG. 8 illustrates a calibration circuit having a ROM in accordance with a preferred embodiment of the invention.

## DETAILED DESCRIPTION OF THE PREFERRED EMBODIMENTS

A detailed description of intra vascular catheters is not given herein, for the features of different types of catheters, namely flow-directed pulmonary artery catheters, left ventricular angiography catheters, and the like are well known to those familiar to the art. Some unique features of such catheters are described by way of example in U.S. Patent Nos. 3,746,003; 3,634,924; 3,995,623; 4,696,304; 4,718,423; and 4,721,115.

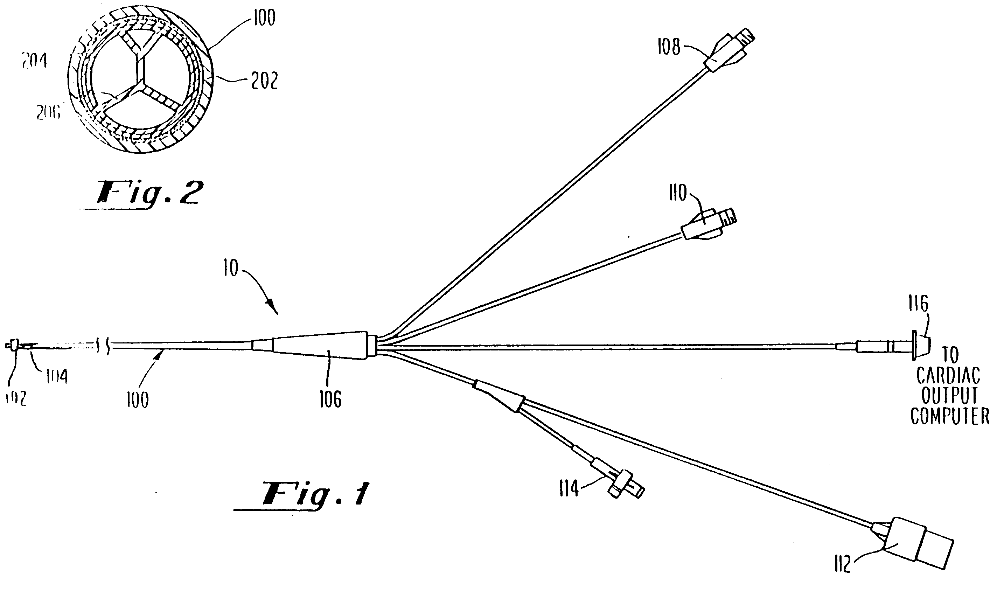

FIG. 1 illustrates a proximal end of a catheter arrangement 10 in accordance with a first embodiment of the invention. As shown, the catheter arrangement 10 comprises a flexible catheter body portion 100 which is adapted for insertion into a blood vessel of a patient and is formed of a non-toxic material such as polyvinyl chloride (PVC). The catheter body portion 100 is also preferably coated with heparin to prevent blood clot formation. At a distal tip of the catheter body portion 100, an inflatable balloon 102 is provided for a flow-directed measurement so that the catheter arrangement 10 may be inserted into the right ventricle of the heart using the customary flow-directed insertion technique. Within a couple of centimeters of the balloon 102 is disposed a temperature sensing device such as a thermistor or thermocouple 104 for measuring the temperature of the flowing blood. This measurement is then used in the thermodilution volumetric blood flow calculation in accordance with known techniques. As shown in FIG. 1, the catheter body portion 100 for insertion into the blood vessel preferably has a length of, for example, 112 centimeters so that it is long enough to be "floated" into the right ventricle of the patient's heart using the flow-directed insertion technique. Insertion may thus be accomplished at bedside without the requirement of fluoroscopy.

At a proximal end of the catheter body portion 100 is provided a catheter body junction 106 through which devices such as a PA distal lumen hub 108, a proximal injectate lumen hub 110, a thermistor or thermocouple connector 112, a balloon inflation valve or stopcock 114, and a heater connector 116 may be inserted into respective filament lead lumens of the catheter body portion 100. In particular, as shown in FIG. 2, the catheter body portion 100 may comprise an outer layer 202 and an intermediate layer 204 which adheres the outer layer 202 to body wall portion 206 of catheter body portion 100. As shown, body wall portion 206 separates the internal area of catheter body portion 100 into one or more lumens for accepting the peripheral devices 108-116. As will be appreciated by those skilled in the art from the following description, one of the lumens permits leads from heater connector 116 to communicate with a downstream heating filament disposed within or about the catheter body portion 100. Although multiple lumens are shown, there is no reason that different leads cannot share a common lumen.

The heater connector 116 communicates with a cardiac output computer so as to receive power signals for controlling the heating filament. Connector 112 forwards temperature changes measured by the thermistor or thermocouple 104 back to the cardiac output computer for calculation of the cardiac output in accordance with a known thermodilution technique.

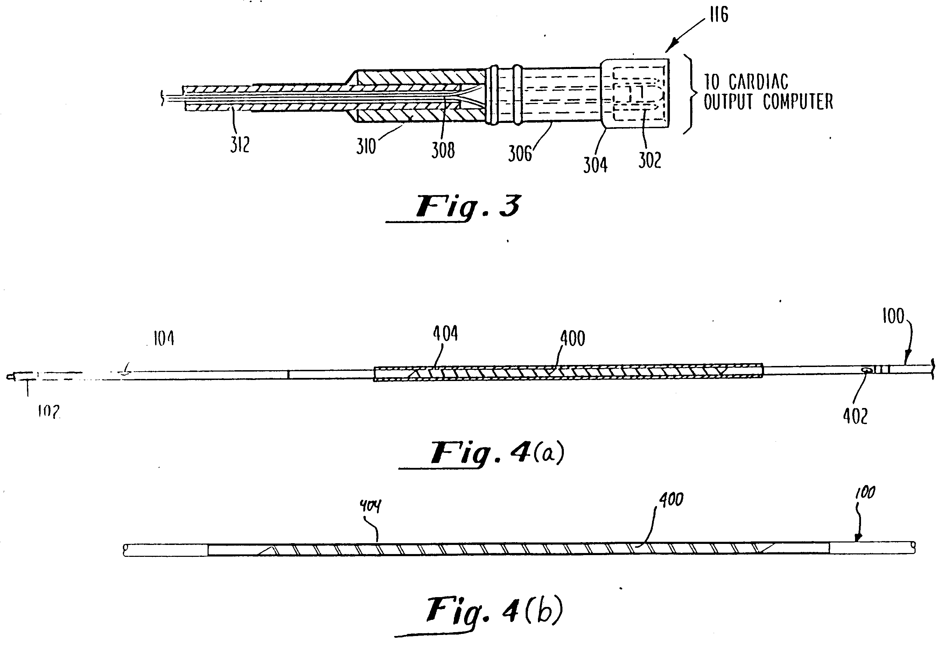

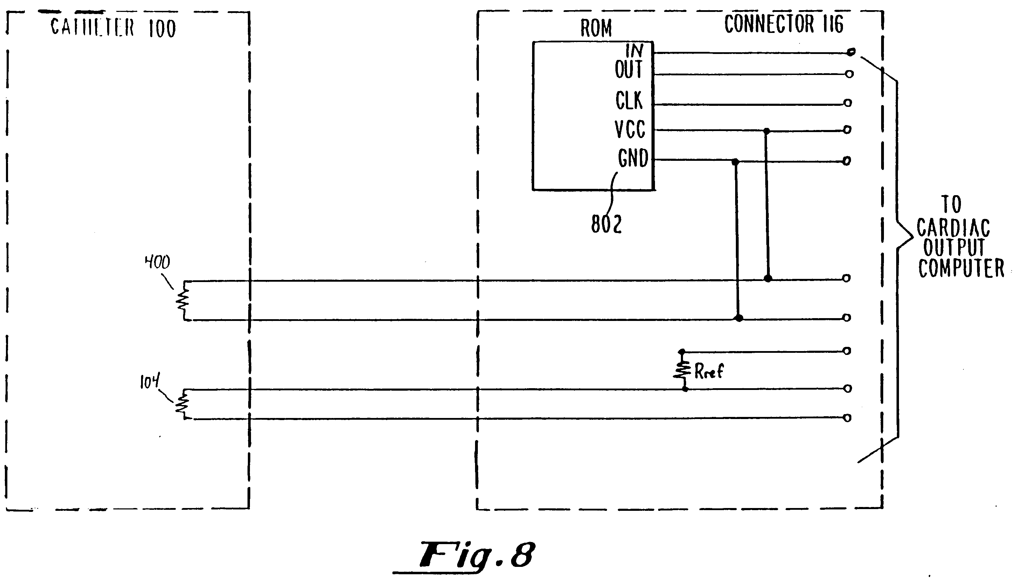

The heater connector 116 is shown in more detail in FIG. 3. As shown, heater connector 116 comprises electrical connector 302 within a plug portion 304 for electrically communicating with the cardiac output computer. The electrical connector 302 communicates through electrical connections in casing 306 with heater wire leads 308. Heater wire leads 308 transverse the length of the support casing 310 and the supporting sheath or heater wire lumen 312 so as to electrically communicate with the heater filament as will be described below. The supporting sheath 312 is preferably made of teflon so as to be flexible yet strong. The supporting sheath 312 supporting the heater wire leads 308 is inserted into a lumen of the catheter body portion 100 to facilitate electrical connection to the heating element. Electrical leads may be similarly "fished" through a lumen to connect to thermistor or thermocouple 104. A more complex connector will be described below with respect to FIG. 8.

FIG. 4(a) illustrates the manner in which the heating filament 400 is wrapped about the outer layer 202 of the catheter body portion 100 in accordance with a first embodiment. As shown, the heating filament 400 is formed so as to be very thin and flat so that it can be wrapped in a non-overlapping manner about the outer layer 202. As shown, an injectate or pacing port 402 may also be provided proximal to heating filament 400. The heating filament 400 is preferably wrapped to extend approximately 5 to 10 centimeters along the outer layer 202 and is disposed so as to be approximately 14 centimeters from the distal tip having balloon 102 of the catheter body portion 100. The heating filament 400 is then surrounded by a thin outer sheath 404 to prevent the heating filament 400 from directly contacting the patient's blood.

Generally, the heating filament 400 is printed on a substrate as a sandwich. The substrate of the heating filament consists of a thin material that is capable of being incorporated into a filament material which is preferably flexible and has the ability to bond with an adhesive. It must also have good heat transfer properties which allow for the conduction of the filament generated heat to the exterior of the outer sheath 404 so as to be applied to the blood. An additional layer of material with high thermal conductivity (e.g., metal foil) may be added to the heater sandwich to help create a more uniform surface temperature. The filament materials of the invention include, but are not limited to, Mylar (R.T.M.) and Kapton (R.T.M.). The filament material, which is adhered to the substrate, must have a high temperature coefficient of resistivity, i.e. greater than $0.001 \ \Omega/\Omega\text{-}°C$, and low thermal capacitance and high thermal conductivity. The material must be capable of being incorporated into the filament substrate and must be capable of being fabricated in thin layers so as to form a sandwich (e.g. Kapton - adhesive - filament metal - adhesive - Kapton). Alloys for the filament material include, but are not limited to, an alloy of 70% nickel and 30% iron or an alloy of 29% nickel, 17% cobalt and 54% iron.

An adhesive material must be selected which is capable of binding to both the outer sheath 404 and the catheter body portion 100, and to the filament substrate, or in some applications, directly to the filament material. The adhesive must be capable of being applied in a thin, even layer, must be non-toxic, must not weaken with time, must tolerate heat from the filament, must tolerate continual flexing, and must bind well in a wet environment (i.e., blood). Such adhesives include, but are not limited to, pressure sensitive adhesives such as Densil (R.T.M.)

In another embodiment, the adhesive, the outer sheath material and the electrical resistive components may all be incorporated into one material. The electrical leads are then connected to the material, which is formed as a sheath or wrapping material and applied directly to the outer layer 202 of the catheter body portion 100 or incorporated during the manufacturing process directly into the outer layer 202 of the catheter body portion 100.

The thin heating filament materials of the invention may be spirally wound around the catheter body portion 100 to form a heating filament 400 as just described. Although the filament substrate or filament heater material may be exposed directly to the blood environment as in the prior art devices, in accordance with the invention the filament substrate and/or filament material are enclosed, surrounded by, or incorporated within an outer sheath 404 for assuring that fragments of filament or filament substrate do not become dislodged into the blood environment. Moreover, by providing a covering material or outer sheath 404, the exterior of the catheter may be made smoother and hence more comfortable for the patient during insertion into the blood vessel. Of course, this structure is made possible because the above-mentioned heater filament material may be formed into a very thin filament which may be non-overlappingly wound about the catheter body portion 100. However, the sheath 404 must also be very thin and flexible and is preferably an adhesive applied by any of a number of techniques over the filament or filament substrate. Such adhesives include, but are not limited to, Master Bond EP37 (T.M.). The resulting catheters are then preferably coated with heparin to prevent blood clot formation.

Regardless of the type of filament material used or the number of layers of materials or sandwich composition, the catheter body may be reduced in diameter in the region where the filament sandwich is wound, as shown in FIG. 4(b). The reduction in catheter body diameter is made such that when the filament sandwich is added, the resulting total diameter in the region of the heating filament is equivalent to the diameter of the adjacent catheter body portion without the filament material. This achieves a uniform transition to the region of the catheter filament, thereby eliminating problems associated with insertion, removal and thrombus formation in regions of irregularities.

A particularly attractive method for applying the sheath 404 is to use a flexible sheath material which can be applied over the filament, filament substrate, and filament-to-catheter body adhesive. Preferably, a material is used which has an appropriate modulus of elasticity and elongation. The material may be fabricated by a technique such as extrusion so that its resting lumen diameter is less than that of the catheter filament subassembly. The sheath material or "tube" may then be expanded using a "vacuum expander" to a size larger than the catheter and attached filament sub-assembly. The catheter and sub-assembly may then be passed into the vacuum expander containing the expanded sheath, positioned in place, and then the vacuum released. The sheath then shrinks, reduces or collapses around the filament sub-assembly so as to maintain a certain tension with the underlying components. Preferably, the vacuum expander contains a chamber which allows for the placement of the sheath material so that the ends of the sheath material may be secured to form a closed chamber between the outer wall and ends of the sheath material and the surrounding chamber. The chamber dimensions may be such as to allow for the expansion of the sheath to a size which is large enough to accept the passage of the catheter body portion 100 and the attached filament sub-assembly. The sheath then may be expanded by applying a vacuum to the chamber and/or positive air pressure to the inside of the sheath. Expansion of the sheath may also be improved by applying heat to the expansion chamber. Conversely, a blow molding technique may be used in accordance with known techniques. A material which may be manufactured to have such a thin wall, an appropriate modulus of elasticity, and an appropriate elongation includes, but is not limited to, Tecoflex™.

Another method of sheath application utilizes shrink material. The sheath may thus be fabricated to be slightly larger than the catheter body portion 100 and the attached filament sub-assembly. It is then applied without the vacuum expander, and when the sheath material is situated in the proper location, it is reduced in size by the application of heat. Again, the proper wall thickness and beginning dimensions are chosen such that following the reduction in size, appropriate tension is maintained with respect to the underneath filament sub-assembly.

Preferably, as described above, the cylindrical heating filament 400 is approximately 10 centimeters in length and is wrapped about the outer wall 202 of the catheter body portion 100 beginning distally about 15 centimeters from the distal tip of the catheter. Then, when the catheter is positioned with the distal tip in the pulmonary artery during a flow-directed measurement, a proximal fluid infusion port of the catheter will lie in the right atrium of the heart or superior vena cava while the distal fluid infusion port will lie in the right ventricle.

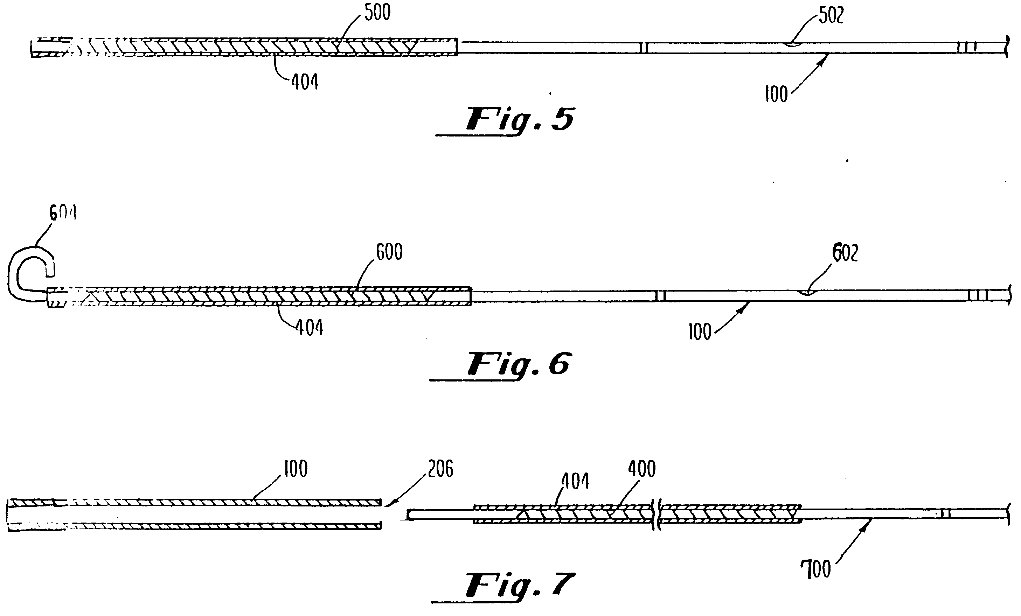

An alternative embodiment of the invention for measuring blood flow in a "retro grade" fashion, such as in the hepatic vein, is shown in FIG. 5. As shown, the heating filament 500 and the thermistor or thermocouple 502 are in reversed positions on the catheter body portion 100 because of the reversed blood flow direction. Since this type of catheter is inserted into the blood vessel against the blood flow, insertion generally requires the use of fluoroscopy for directing the catheter into place for measurement. Since the embodiment of FIG. 5 is not a flow-directed catheter, a balloon at the distal tip is not used.

The alternative embodiment of FIG. 6 may also be used for measuring blood flow in a "retro grade" fashion, as in the left ventricle of the heart, whereby the heating filament 600 and thermistor or thermocouple 602 are in reversed positions on the catheter body portion 100 as in the embodiment of FIG. 5. As in the FIG. 5 embodiment, insertion generally requires fluoroscopy and a balloon tip is not used. However, a pigtail tip 604 is preferably used in this embodiment to prevent vessel rupture.

During operation, since the heating filament formed as described above is used primarily to insert heat into the blood stream, it will rise to a temperature higher than the surrounding environment. Thus, it is necessary to know the filament temperature since, should the temperature become excessive, damage could result to the surrounding blood and tissues. Normally, a second temperature sensing device such as a thermistor or thermocouple would need to be embedded next to the filament to measure its temperature. However, by using a filament material which has a high temperature coefficient of resistance as herein described, not only can it be used as a heat supplier, but it can also serve as its own temperature sensing device. For example, resistance of any material is measured as follows:

$$R = \frac{\rho \cdot \ell}{A},$$

where    $\rho$ is the resistivity,
         $\ell$ is the length, and
         A is the cross-sectional area.

Then:

$$\Delta R = \frac{\Delta\rho \cdot \ell}{A},$$

and if $\alpha$, the mean temperature coefficient of resistivity, is defined as:

$$\alpha = \frac{1}{\rho} \cdot \frac{\Delta\rho}{\Delta T},$$

where    $\Delta\rho$ is the change in the coefficient and
         $\Delta T$ is the change in temperature,

then:

$$\Delta T = \Delta R \frac{A}{\ell \cdot \alpha \cdot \rho}.$$

Then, by measuring the current (i) and the voltage (v), both delivered power and resistance of the filament can be simultaneously measured as:

$$\frac{\Delta v}{\Delta i} = \Delta R.$$

The heating filament 400 of the invention typically consists of a cylindrical design which is approximately 5-10 centimeters in length. Heater wire leads 308 are attached to the heating filament 400, and the heating filament 400 is placed at the desired distance from the thermistor or thermocouple 104 (10 cm in FIGS. 4(a) and (b)). Then, as previously described, the heat transfer is such that the heat passes from the heater filament 400 through the outer sheath 404 into the blood. Of course, the heating filament 400 must be flexible such that it does not increase the stiffness of the catheter body portion 100.

In accordance with another embodiment of the invention, as shown in FIG. 7, the heating filament 400 may be made as a mobile module supported by a flexible supporting member 700 which can be inserted or withdrawn from the catheter lumen after the catheter has been inserted into the patient. This has the advantage that the catheter can be inserted into the patient when it is not known whether measurement of blood flow is required. Should the measurement of blood flow become desirable, the mobile filament module can be inserted and the measurement started. This feature of the invention is particularly helpful in a clinical setting, for although pulmonary artery catheters were originally designed to measure distal pressure, more features have been added such as bolus thermodilution cardiac output measurements, cardiac pacing and mixed venous saturation. Thus, the clinical problem now is to know which catheter to use, for not all patients require all measurement modalities.

The design is thus as a pulmonary artery catheter which has one or more ports and/or lumens which will accept the particular modules (as shown in FIG. 1) for a particular measurement modality. For example, for a 4-lumen catheter of the type shown in cross-section of FIG. 2, one lumen may be dedicated to measuring distal catheter pressure, one lumen dedicated for distal balloon inflation and passage of two distal thermistor or thermocouple leads, and one lumen dedicated to proximal fluid infusion while the fourth is left open. Moreover, another lumen may receive a module for measuring mixed venous oxygen saturation including a fiber optic bundle. Other modules may be designed at the user's discretion.

During use, the pulmonary artery catheter (with the vacant lumen) is inserted in the usual and customary fashion. After insertion, if so desired, the physician or the user may electrically pace the heart by passing a modulus or wire through the vacant catheter lumen so as to connect the proximal end of the wire to the appropriate electronics. Such a concept of a removable pacing wire has been previously described by Swendson, et al. in U.S. Patent No. 4,759,378, for example. On the other hand, if the measurement of mixed venous saturation is desired, the pacing wire modules would be removed and a fiber optics modulus inserted in the vacant lumen for measuring mixed venous saturation, and the proximal end of the fiber optics would be attached to the appropriate electronics. Such fiber optics techniques for measuring mixed venous saturation are described by Willis, et al. in U.S. Patent No. 4,718,423, for example. However, the fiber optics technique taught by Willis, et al. is not removable; therefore, if cardiac output is desired, the vacant lumen must be replaced with the thermal transducer filament or other apparatus modulus for performing cardiac output measurement. Of course, the scope of the invention is not limited to just these modalities, but to any modalities which could be used at the user's discretion.

Thus, the heating filament 400 is placed either around the catheter body portion 100 but within an outer sheath 404 or is placed within the catheter body portion 100, namely, in a lumen thereof. In either case, the heating filament 400 does not directly contact the patient's blood. This is in marked contrast to previous embodiments whereby the heating elements are generally placed on the exterior of the catheter or the filaments are used as unattached free-floating pieces. Instead, the heating filament 400 is placed such that the heat transfer properties of the catheter body portion 100, the outer sheath material and heating filament material allow the transmission of heat to the exterior environment, namely, the blood stream. Such an arrangement has significant implications since an internally placed heating filament reduces the probability of harmful blood clot formation, electrical leakage currents, or unusually high filament blood contact temperatures.

When a thermodilution catheter in accordance with the invention is connected to a cardiac output computer via heater connector 116, an electrical current is applied to the heating filament in the form of pulses. When the heating filament is activated, an approximate average of 7.5 watts of power may be delivered to the heating filament. During operation, as described above, the cardiac output computer may continuously measure and monitor the filament temperature so as to limit the peak filament temperature to a maximum of 52°C (which corresponds to a peak surface temperature of about 48°C and an average surface temperature of about 44°C, depending upon the material composition and thickness). For example, in the event the heating filament temperature exceeds 52°C for more than, say, 15 seconds at full power, the delivered heating filament power is reduced. Then, if the heating filament temperature exceeds 52°C for more than, say, 15 seconds at reduced power, the heating filament power may be shut off and a panel alarm activated. This prevents the peak surface temperature from exceeding 48°C. Moreover, the average catheter surface temperature should not exceed 44°C since the power will be switched "ON" approximately 50% of the time. Furthermore, if the average cardiac output exceeds 3.5 liters/minute, the catheter's average surface temperature will generally remain below 44°C. Thus, regulation of power to the catheter only becomes an issue when the cardiac output becomes less than about 3.5 liters/minute. However, since the power to the heating filament is reduced or shut off as the filament temperature reaches 52°C, the heating element of the invention can be made relatively fail-safe through closed-loop control of the surface temperature.

By using a power source which is a constant voltage source, an increasing catheter filament temperature can be directly detected as an increasing filament resistance which reduces the power delivered to the heating filament. In this manner, the actual current and voltage to the catheter filament may be continuously monitored. From the values of current and voltage, a delivered power may be calculated which is needed to calculate flow, and the filament resistance is calculated and used for computing the filament temperature. Thus, at all times, the actual filament temperature is known. Preferably, the following algorithm is followed to insure that the filament temperature remains within safe limits:

(1) When the cardiac output computer starts, the delivered power to the heating filament is maintained at approximately 4 watts average power.
(2) The filament temperature is monitored for several seconds.
(3) If the peak filament temperature has not exceeded 46°C, the filament power is increased to an average power of 7.5 watts.
(4) If at any time the peak filament temperature exceeds 48°C for more than, say, 15 seconds, the delivered filament power is reduced.
(5) If at any time the average delivered filament power is approximately 4 watts and the peak filament temperature exceeds 48°C for more than, say, 15 seconds, the computer shuts off and displays an error message.

The cardiac output may be measured continuously by turning the heating filament on and off in a predetermined pattern and generating a characteristic thermodilution curve by mathematical process.

By using an indicator dilution method in accordance with a stochastic system, cardiac output may be measured in a noisy environment even when a small heat input source as herein described is used. The stochastic techniques of the type described in the afore-mentioned application are different from classical empirical techniques in that the input signal or energy is applied over a period of time and the nature of the statistical properties of the input and output signals are of interest. Thus, during operation in accordance with this technique, the supplied heat will produce a small temperature change in the flowing blood which is detected at the distal thermistor or thermocouple 104. Through a mathematical procedure known as cross-correlation, a scaled characteristic thermodilution "wash-out" curve is reconstructed. The cardiac output may then be calculated by measuring the area under this "wash-out" curve if the amount of heat delivered to the blood by the heating filament is also known.

In the calculation of cardiac output using such thermodilution techniques, it is necessary to know certain properties about the measuring transducer, such as the thermistor or thermocouple 104, and the heat application or heating filament efficiency, for in the manufacturing process it is difficult to produce either thermistors or thermocouples 104 or heating filaments 400 which uniformly have the same properties. Thus, to reduce the errors which would be introduced into the calculation of cardiac output due to these variances, it is necessary to calibrate or measure the physical properties of both the thermistor or thermocouple 104 and the heating filament 400. Since in a clinical environment each cardiac output computer may be attached over time to various pulmonary artery catheters and to eliminate the need for the user to manually transcribe these calibration numbers to the computer, a coding technique has been developed to pass the calibration information.

Prior art thermodilution catheters and pulse oximeter sensors have used resistors to code the values for thermistors or LEDs. For example, New et al. in U.S. Patent No. 4,700,708 use a resistor to calibrate LED wavelengths on a pulse oximeter. Calibration of the heating element may be conducted by measuring the heater resistance at a known temperature. The catheter assembly can then use the previously calibrated thermistor or thermocouple and a built-in ohm meter to establish a calibrated reference point for the heater element. This approach has the advantage of calibrating the heater immediately prior to use in a patient at the patient's body temperature. Such an accurate calibration of heater resistance and temperature is necessary to accurately monitor heater temperature to insure patient safety.

The calibration circuit may include passive electronic components such as resistors, inductors and capacitors such that the value of the components correspond to a particular calibration value or number according to a predetermined table. On the other hand, active electronic components including numerous nonlinear components may be used such that a particular performance corresponds to a particular calibration number or value. Such calibration information is preferably stored in a memory component such as a ROM (Read Only Memory), RAM (Random Access Memory), nonvolatile memory devices or other types of memory or digital devices. The calibration information preferably includes codes that represent the filament resistance, filament efficiency, and other parameters. If properly selected, one or more electronic components may be used to encode the calibration information of the thermistor or thermocouple, such as its $\beta$ value, and the filament resistance, filament efficiency and other parameters.

Thus, the calibration information for both the thermistor or thermocouple 104 and the heating filament 400 may be encoded by one or more active or passive electronic components or these values may be stored in a suitable memory device. The cardiac output computer may then decode this information and incorporate it into the calculation of cardiac output. However, this step may be eliminated if the actual appropriate software is contained in the catheter itself. For example, a memory device such as a ROM may be contained in the catheter with a portion of the software utilized by the cardiac output computer resident within it. Such information might include program segments or historical patient data. Thus, when the catheter is connected to the cardiac output computer, prior to the beginning of processing for determining the cardiac output, the software or program segment contained in the catheter memory device (ROM) may be transferred to the main software program of the cardiac output computer. This feature also provides an additional safety feature, for the cardiac output computer will not start until it has transferred the program segment and incorporated this segment into its own program.

The calibration circuitry of the type just described can be seen by way of example in FIG. 8. As should be apparent to one of ordinary skill in the art, the calibration circuit of FIG. 8 is quite different from that used in typical prior art thermodilution catheters. In particular, classic thermodilution catheters use calibration resistances which are connected in series with the thermistor or thermocouple. In such devices, the reference resistor is calibrated to match the thermistor or thermocouple for a standard temperature. In this manner, compensation for variability in the thermistors or thermocouples may be achieved. However, by using the calibration circuit whereby a ROM containing calibration data is included within the connector of the catheter, such a reference resistor for calibration purposes is not needed. Such a ROM is shown as ROM 802 of connector 116 in FIG. 8.

Preferably, the software module referred to above is stored in the ROM 802 and includes such things as the format version for the calibration data, trademark information, historical patient data (such as cardiac output for the previous several hours) or whatever information is desired for controlling the cardiac output program. Thus, by placing the encoded calibration data within the ROM 802 and placing the ROM 802 on the catheter, the thermistor or thermocouple reference resistance may be eliminated. In addition, only a catheter having a ROM 802 storing the necessary information for operating the program of the cardiac output computer may be used in conjunction with the cardiac output computer to obtain the desired calculation.

Although a number of exemplary embodiments of the invention have been described in detail above, those skilled in the art will readily appreciate that many additional modifications are possible. For example, rather than wrapping the heating element 400 around the catheter body portion 100, the heating element may be included in the body wall portion 202 of the catheter body portion 100. In addition, the heating element 400 may be made in multiple contiguous sections, whereby by measuring the temperature of each section it is possible to determine whether one section is malfunctioning. Such malfunctions could be due to filament abnormalities or due to physiologic aberrations such as clotting. The discrepancy in temperature would alert the user to a potential problem. However, such a section arrangement would require additional electrical leads, and the catheter would need to be modified accordingly. Alternatively, the heating filament may be used in conjunction with a guide wire for angioplasty, where the thermistor or thermocouple will be miniaturized and placed on the guide wire, and the heater placed upstream on the guide wire. The resulting device may then be inserted into a catheter lumen of the type described herein. In addition, the heating filament may be placed ahead of or behind balloon 102 as desired.

## Claims

1. A thermodilution catheter apparatus, comprising a flexible tubular catheter member (10) adapted for introduction into a blood vessel of a patient, a flexible beating filament (400) for applying a predetermined quantity of heat to blood in said blood vessel and disposed with respect to said catheter member (10) so as not to touch the patient's blood when said catheter member (100) is inserted into the blood vessel, and temperature detecting means (104,602) for detecting upstream or downstream temperature variations of said blood as a result of application of said predetermined quantity of heat to said blood;

   characterised in that the heating filament (400) is made of a metal alloy having a temperature coefficient of resistivity greater than 0.001 $\Omega/\Omega$-°C, resistance of said heating filament being proportional or inversely proportional to its temperature.

2. An apparatus as in Claim 1, further including means to reduce power to said heating Filament (400,600) when resistance of said heating filament exceeds a predetermined resistance amount.

3. An apparatus as in Claim 2, wherein said power reduction means is adapted to reduce the power to said heating filament when the temperature of said heating filament reaches approximately 52°C.

4. An apparatus as in any preceding claim, wherein said heating filament (400) is comprised of approximately 70% nickel and 30% iron.

5. An apparatus as in Claim 1, 2 or 3, wherein said heating filament (400) is comprised of approximately 29% nickel, 17% cobalt and 54% iron.

6. An apparatus as in any preceding claim, wherein said catheter member (10) comprises a substantially cylindrical body wall portion (100) and an outer sheath (404), said heating filament (400) being wrapped in a thin layer about said body wall portion and enclosed by said sheath.

7. An apparatus as in Claim 6, further comprising a layer of material with a high thermal conductivity disposed about said heating filament (400) so as to create a more uniform surface temperature.

8. An apparatus as in Claim 6 or 7, wherein said body wall portion (100) has a reduced diameter in a region where said heating filament (400) is wrapped therearound such that the resulting total diameter in said region is approximately equal to the diameter of said body wall portion in other regions.

9. An apparatus as in Claim 6, 7 or 8, wherein said sheath (404) is a flexible material formed by one of extrusion and blow molding.

10. An apparatus as in Claim 6, 7 or 8, wherein said sheath (404) is a flexible material which shrinks to form-fit said heating filament (400) and said body wall portion (100) when a sufficient quantity of heat is applied thereto.

11. An apparatus as in any preceding claim, wherein said temperature detecting means (104) comprises one of a thermistor and thermocouple approximate to a distal end of said catheter member (10) and disposed distally with respect to said heating filament (400).

12. An apparatus as in any one of Claims 1 to 10, wherein said temperature detecting means (602) comprises one of a thermistor or thermocouple disposed proximally with respect to said heating filament (600).

13. An apparatus as in Claim 12, wherein a distal end of said catheter member comprises a pigtail tip (604).

14. An apparatus as in ally preceding claim, further comprising at least one set of electrical leads (308) attached to said heating filament (400) at a first end thereof so as to apply power to said heating filament.

15. An apparatus as in Claim 14, wherein the opposite end of said set of electrical leads (308) is connected to a cardiac output computer which applies an appropriate power signal to said electrical leads when said predetermined quantity of heat is to be applied to said blood.

16. An apparatus as in Claim 1, wherein said catheter member (10) has at least one lumen through which said heating filament (400) is removably inserted.

17. An apparatus as in Claim 16, wherein said heating filament (400) is wrapped in a thin layer about a substantially cylindrical supporting member (700), said supporting member and thin layer having a combined outer diameter which is approximately equal to an inner diameter of said at least one lumen.

18. An apparatus as in Claim 17, wherein said heating filament (400) is approximately 5 to 10 centimeters in length and is disposed approximately 10 centimeters from said temperature detecting means (104) during measurement.

19. An apparatus as in Claim 1, further comprising calibrating means for calibrating both said heating filament (400,600) and said temperature detecting means (104).

20. An apparatus as in Claim 19, wherein said calibrating means comprises a memory contained at a proximal end of said catheter member (10) for storing calibration information for either said heating filament or both said heating filament (400,600) and said temperature detecting means (104).

21. An apparatus as in Claim 20, wherein said calibration information includes heating filament resistance at a given temperature, heating filament heat transfer efficiency, temperature coefficient of resistivity and thermistor or thermocouple information.

22. An apparatus as in Claim 21, wherein said memory is connected to a cardiac output computer and said memory further stores a program segment of a program used by said cardiac output computer to calculate cardiac output of said patient, whereby calculation of the patient's cardiac output cannot commence until said cardiac output computer is connected to said memory and said program segment is transferred to said cardiac output computer.

**Patentansprüche**

1. Thermodilutions-Kathetervorrichtung, umfassend einen flexiblen röhrenförmigen Katheterkörper (10) zur Einführung in ein Blutgefäß eines Patienten, einen flexiblen Heizdraht (400) zur Applikation einer bestimmten Wärmemenge an das Blut in dem Blutgefäß, der in Bezug auf den Katheterkörper (10) so angeordnet ist, daß er nicht mit dem Blut des Patienten in Kontakt kommt, wenn der Katheterkörper (10) in das Blutgefäß eingeführt wird, und Temperaturmeßelemente (104, 602) zur Bestimmung der stromaufwärtigen oder stromabwärtigen Temperaturänderungen des Bluts als Ergebnis der Applikation der bestimmten Wärmemenge an das Blut; dadurch gekennzeichnet, daß der Heizdraht (400) aus einer Metallegierung besteht, die einen Temperaturkoeffizienten des spezifischen Wiederstandes besitzt, der größer ist als 0,001 $\Omega/\Omega$-°C, wobei der Widerstand des Heizdrahtes proportional oder umgekehrt proportional zu seiner Temperatur ist.

2. Vorrichtung nach Anspruch 1, dadurch gekennzeichnet, daß sie außerdem eine Einrichtung zur Verringerung der an den Heizdraht (400, 600) angelegten elektrischen Energie aufweist, wenn der Widerstand des Heizdrahtes einen bestimmten Widerstandswert übersteigt.

3. Vorrichtung nach Anspruch 2, dadurch gekennzeichnet, daß die Einrichtung zur Verringerung der elektrischen Energie so ausgestaltet ist, um die an den Heizdraht angelegte elektrische Energie zu verringern, wenn die Temperatur des Heizdrahtes ca. 52 °C erreicht.

4. Vorrichtung nach einem der vorhergehenden Ansprüche, dadurch gekennzeichnet, daß der Heizdraht (400) aus ca. 70 % Nickel und 30 % Eisen besteht.

5. Vorrichtung nach Anspruch 1, 2 oder 3, dadurch gekennzeichnet, daß der Heizdraht (400) aus ca. 29 % Nickel, 17 % Kobalt und 54 % Eisen besteht.

6. Vorrichtung nach einem der vorhergehenden Ansprüche, dadurch gekennzeichnet, daß der Katheterkörper (10) einen im wesentlichen zylindrischen Wandteil (100) und eine äußere Umhüllung (404) aufweist, wobei der Heizdraht (400) in einer dünnen Schicht um den Wandteil gewunden ist, und von der Umhüllung umschlossen wird.

7. Vorrichtung nach Anspruch 6, dadurch gekennzeichnet, daß sie außerdem eine Schicht aus einem Material mit einer hohen thermischen Leitfähigkeit aufweist, die um den Heizdraht (400) angeordnet ist, um eine gleichmäßigere Oberflächentemperatur auszubilden.

8. Vorrichtung nach Anspruch 6 oder 7, dadurch gekennzeichnet, daß der Wandteil (100) in einem Bereich, in dem der Heizdraht (400) darum gewunden ist, einen verringerten Durchmesser besitzt, damit der resultierende Gesamtdurchmesser in diesem Bereich etwa gleich dem Durchmesser des Wandteils in anderen Bereichen ist.

9. Vorrichtung nach Anspruch 6, 7 oder 8, dadurch gekennzeichnet, daß die Umhüllung (404) aus einem flexiblen Material besteht und durch Strangpressen oder Blasformen ausgebildet wurde.

10. Vorrichtung nach Anspruch 6, 7 oder 8, dadurch gekennzeichnet, daß die Umhüllung (404) aus einem flexiblen Material besteht, das sich dem Heizdraht (400) und dem Wandteil (100) formschlüssig anpaßt, wenn darauf eine ausreichende Wärmemenge appliziert wird.

11. Vorrichtung nach einem der vorhergehenden Ansprüche, dadurch gekennzeichnet, daß das Temperaturmeßelement (104) ein solches ist ausgewählt aus einem Thermistor und Thermoelement, das sich in der Nähe eines distalen Endes des Katheterkörpers (10) befindet und in Bezug auf den Heizdraht (400) distal angeordnet ist.

12. Vorrichtung nach einem der Ansprüche 1 bis 10, dadurch gekennzeichnet, daß das Temperaturmeßelement (602) ein solches ist ausgewählt aus einem Thermistor oder Thermoelement, das in Bezug auf den Heizdraht (600) proximal angeordnet ist.

13. Vorrichtung nach Anspruch 12, dadurch gekennzeichnet, daß ein distales Ende des Katheterkörpers eine Anschlußlitze (604) aufweist.

14. Vorrichtung nach einem der vorhergehenden Ansprüche, dadurch gekennzeichnet, daß sie außerdem mindestens einen Satz elektrische Zuleitungen (308) aufweist, die mit dem Heizdraht (400) an einem ersten Ende davon verbunden sind, um den Heizdraht mit elektrischer Energie zu versorgen.

15. Vorrichtung nach Anspruch 14, dadurch gekennzeichnet, daß das entgegengesetzte Ende dieses Satzes elektrischer Zuleitungen (308) mit einem Herzleistungs-Computer verbunden ist, der an die elektrischen Zuleitungen eine geeignete Leistung abgibt, wenn die bestimmte Wärmemenge dem Blut zugeführt werden soll.

16. Vorrichtung nach Anspruch 1, dadurch gekennzeichnet, daß der Katheterkörper (10) mindestens ein Lumen aufweist, durch das der Heizdraht (400) entfernbar eingeführt ist.

17. Vorrichtung nach Anspruch 16, dadurch gekennzeichnet, daß der Heizdraht (400) in einer dünnen Schicht um einen im wesentlichen zylindrischen Stützkörper (700) gewunden ist, wobei der Stützkörper und die dünne Schicht einen kombinierten Außendurchmesser aufweisen, der etwa gleich dem Innendurchmesser dem mindestens einen Lumens ist.

18. Vorrichtung nach Anspruch 17, dadurch gekennzeichnet, daß der Heizdraht (400) ca. 5 bis 10 cm lang ist und während der Messung ca. 10 cm von dem Temperaturmeßelement (104) entfernt angeordnet ist.

19. Vorrichtung nach Anspruch 1, dadurch gekennzeichnet, daß sie außerdem einen Kalibrator zur Kalibrierung des Heizdrahtes (400, 600) und des Temperaturmeßelements (104) aufweist.

20. Vorrichtung nach Anspruch 19, dadurch gekennzeichnet, daß der Kalibrator einen Speicher aufweist, der an einem proximalen Ende des Katheterkörpers (10) zur Speicherung von Kalibrierungsinformationen für den Heizdraht oder den Heizdraht (400, 600) und das Temperaturmeßelement (104) enthalten ist.

21. Vorrichtung nach Anspruch 20, dadurch gekennzeichnet, daß die Kalibrierungsinformationen den Widerstand des Heizdrahtes bei einer bestimmten Temperatur, die Wärmeübertragungseffizienz des Heizdrahtes, den Temperaturkoeffizienten des spezifischen Widerstandes und Information über den Thermistor oder das Thermoelement umfassen.

22. Vorrichtung nach Anspruch 21, dadurch gekennzeichnet, daß der Speicher mit dem Herzleistungs-Computer verbunden ist und der Speicher außerdem ein Programmsegment eines Programmes speichert, das von dem Herzleistungs-Computer verwendet wird, um die Herzleistung des Patienten zu berechnen, wobei die Berechnung der Herzleitung des Patienten nicht beginnen kann, bevor der Herzleistungs-Computer mit dem Speicher verbunden ist und das Programmsegment an den Herzleistungs-Computer übertragen ist.

## Revendications

1. Dispositif de cathéter de thermodilution, comprenant un élément de cathéter tubulaire flexible (10) prévu pour introduction dans un vaisseau sanguin d'un patient, un filament chauffant flexible (400) pour appliquer une quantité prédéterminée de chaleur au sang dans ledit vaisseau sanguin et disposé par rapport audit élément de cathéter (10) de façon à ne pas toucher le sang du patient lorsque ledit élément de cathéter (100) est inséré dans le vaisseau sanguin, et des moyens de détection de température (104,602) pour détecter les variations de température dudit sang en amont ou en aval comme résultat de l'application de ladite quantité prédéterminée de chaleur audit sang ;
caractérisé en ce que le filament chauffant (400) est fabriqué en un alliage métallique ayant un coefficient de variation de la résistivité en fonction de la température supérieur à 0,001 $\Omega/\Omega$-°C, la résistance dudit filament chauffant étant proportionnelle ou inversement proportionnelle à sa température.

2. Dispositif selon la revendication 1, comprenant en outre des moyens de réduction de la puissance fournie audit filament chauffant (400,600) lorsque la résistance dudit filament chauffant dépasse une valeur de résistance prédéterminée.

3. Dispositif selon la revendication 2, dans lequel lesdits moyens de réduction de puissance sont prévus pour réduire la puissance fournie audit filament chauffant lorsque la température dudit filament chauffant atteint 52°C environ.

4. Dispositif selon une quelconque des revendications précédentes, dans lequel ledit filament chauffant (400) est composé d'environ 70% de nickel et 30% de fer.

5. Dispositif selon la revendication 1,2 ou 3, dans lequel ledit filament chauffant (400) est composé d'environ 29% de nickel, 17% de cobalt et 54% de fer.

6. Dispositif selon une quelconque des revendications précédentes, dans lequel ledit élément de cathéter (10) comprend une partie de paroi de corps sensiblement cylindrique (100) et une gaine extérieure (404), ledit filament chauffant (400) étant enroulé en une couche mince autour de ladite partie de paroi de corps et entouré par ladite gaine.

7. Dispositif selon la revendication 6, comprenant en outre une couche de matière de conductivité thermique élevée, disposée autour dudit filament chauffant (400) de façon à créer une température de surface plus uniforme.

8. Dispositif selon la revendication 6 ou 7, dans lequel ladite partie de paroi de corps (100) a un diamètre réduit dans une région où ledit filament chauffant (400) est enroulé autour du corps, de sorte que le diamètre total résultant dans ladite région est approximativement égal au diamètre de ladite partie de paroi de corps dans les autres régions.

9. Dispositif selon la revendication 6, 7 ou 8, dans lequel ladite gaine (404) est une matière flexible formée par l'un des procédés d'extrusion et de moulage par soufflage.

10. Dispositif selon la revendication 6, 7 ou 8, dans lequel ladite gaine (404) est une matière flexible qui se contracte de manière à s'ajuster à la forme dudit filament chauffant (400) et de ladite partie de paroi de corps (100) lorsqu'on lui applique une chaleur suffisante.

11. Dispositif selon une quelconque des revendications précédentes, dans lequel lesdits moyens de détection de température (104) comprennent l'un ou l'autre d'une thermistance et d'un thermocouple près d'une extrémité distale dudit élément de cathéter (10) et dans une position distale par rapport audit filament chauffant (400).

12. Dispositif selon une quelconque des revendications 1 à 10, dans lequel lesdits moyens de détection de température (602) comprennent l'un ou l'autre d'une thermistance ou d'un thermocouple placé en position proximale par rapport audit filament chauffant (600).

13. Dispositif selon la revendication 12, dans lequel une extrémité distale dudit élément de cathéter comporte un bout en queue de cochon (604).

14. Dispositif selon une quelconque des revendications précédentes, comprenant en outre au moins un ensemble de conducteurs électriques (308) connecté au dit filament chauffant (400) à une première extrémité du dit ensemble, de façon à fournir une puissance audit élément chauffant.

15. Dispositif selon la revendication 14, dans lequel l'extrémité opposée dudit ensemble de conducteurs électriques (308) est connectée à un ordinateur de sortie cardiaque qui applique un signal de puissance appropriée auxdits conducteurs électriques lorsqu'on veut appliquer ladite quantité de chaleur prédéterminée audit sang.

16. Dispositif selon la revendication 1, dans lequel ledit élément de cathéter (10) comporte au moins un passage dans lequel ledit filament chauffant (400) est inséré de façon amovible.

17. Dispositif selon la revendication 16, dans lequel ledit filament chauffant (400) est enroulé en une couche mince autour d'un élément de support sensiblement cylindrique (700), ledit élément de support et ladite couche mince ayant un diamètre extérieur combiné qui est approximativement égal à un diamètre intérieur dudit au moins un passage.

18. Dispositif selon la revendication 17, dans lequel ledit filament chauffant (400) a une longueur de 5 à 10 cm environ et est placé à 10 cm environ desdits moyens de détection de température (104) pendant la mesure.

19. Dispositif selon la revendication 1, comprenant en outre des moyens d'étalonnage pour étalonner à la fois ledit filament chauffant (400,600) et lesdits moyens de détection de température (104).

20. Dispositif selon la revendication 19, dans lequel lesdits moyens d'étalonnage comprennent une mémoire contenue à une extrémité proximale dudit élément de cathéter (10), pour stocker des informations d'étalonnage pour ledit filament de chauffage ou à la fois ledit filament de chauffage (400,600) et lesdits moyens de détection de température (104).

21. Dispositif selon la revendication 20, dans lequel lesdites informations d'étalonnage comprennent la résistance du filament chauffant à une température donnée, le rendement de transfert de chaleur du filament chauffant, le coefficient de variation de la résistivité en fonction de la température et des informations relatives à la thermistance ou au thermocouple.

22. Dispositif selon la revendication 21, dans lequel ladite mémoire est connectée à un ordinateur de sortie cardiaque et ladite mémoire stocke en outre un segment de programme d'un programme utilisé par ledit ordinateur de sortie cardiaque pour calculer la sortie cardiaque dudit patient, de sorte que le calcul de la sortie cardiaque du patient ne peut pas commencer jusqu'à ce que ledit ordinateur de sortie cardiaque soit connecté à ladite mémoire et ledit segment de programme soit transféré audit ordinateur de sortie cardiaque.

Fig.2

Fig.1

TO
CARDIAC
OUTPUT
COMPUTER

EP 0 637 219 B1

Fig. 3

Fig. 4(a)

Fig. 4(b)

**_Fig. 5_**

**_Fig. 6_**

**_Fig. 7_**

EP 0 637 219 B1

CATHETER 100

CONNECTOR 116

ROM

IN
OUT
CLK
VCC
GND

802

400

104

R_ref

TO CARDIAC OUTPUT COMPUTER

Fig.8

EP 0 637 219 B1